# FASCICULE DE BREVET EUROPEEN

(11) **EP 3 113 821 B1**
(45) Date de publication et mention de la délivrance du brevet: **19.08.2020**
(21) Numéro de dépôt: 15757883.2
(22) Date de dépôt: 30.01.2015
(51) Int. Cl.: A61M 16/06, A61M 11/06, A61M 15/00, A61M 16/08, A61M 16/20

(54) **MASQUE FACIAL POUR INHALATION, ADAPTE A L'ADMINISTRATION D'UN GAZ OU D'UNE MOLÉCULE THÉRAPEUTIQUE DANS LE CADRE D'UN TRAITEMENT PAR INHALATION, EN PARTICULIER CHEZ LES ENFANTS**
INHALATIONSGESICHTSMASKE ZUR ABGABE EINES THERAPEUTISCHEN GASES ODER EINES MOLEKÜLS ALS TEIL EINER BEHANDLUNG DURCH INHALATION, INSBESONDERE BEI KINDERN
FACE MASK FOR INHALATION, SUITABLE FOR DELIVERING A THERAPEUTIC GAS OR MOLECULE AS PART OF A TREATMENT BY INHALATION, IN PARTICULAR IN CHILDREN

(30) Priorité: 04.03.2014 FR 1451756
(43) Date de publication de la demande: 11.01.2017
(73) Titulaire: Protecsom Amerique du Nord Inc., Drummondville, Québec J2C 7V3 (CA)
(72) Inventeur: POREE, Thierry, 50330 St-Pierre-Eglise (FR)
(74) Mandataire: Argyma
(86) Numéro de dépôt international: PCT/CA2015/000057
(87) Numéro de publication internationale: WO 2015/131262

(56) Documents cités:
- WO-A1-99/21602
- WO-A1-99/21602
- WO-A1-2010/076715
- DE-C1- 4 004 157
- GB-A- 2 385 533
- US-A- 1 310 825
- US-A- 4 519 399
- US-A- 5 243 972
- US-A- 5 243 972
- US-A- 5 265 595
- US-A- 5 265 595
- US-A1- 2002 170 557
- US-A1- 2008 245 364
- US-A1- 2010 059 058
- US-A1- 2013 255 678

## Description

### BACKGROUND

### (a) Domaine de l'invention

La présente invention concerne un masque facial pour inhalation, adapté à l'administration d'un gaz ou d'une molécule thérapeutique dans le cadre d'un traitement par inhalation, en particulier chez les enfants.

### (b) Description de l'Art Antérieur

Un masque facial pour inhalation est un dispositif médical favorisant l'entrée d'un gaz respiratoire dans l'organisme d'une personne ayant des difficultés temporaires ou permanentes à assumer le réflexe respiratoire. Il peut également être utilisé afin de diffuser une molécule thérapeutique dans l'organisme d'un patient par l'intermédiaire de ses voies respiratoires.

Les voies respiratoires partent du nez et de la bouche et s'étendent jusqu'aux alvéoles pulmonaires. Elles assurent la respiration d'un individu, et sont donc essentielles à sa survie. L'entrée du gaz respiratoire se fait au niveau du nez et/ou de la bouche, et les alvéoles pulmonaires sont le siège des échanges gazeux avec le milieu interne du corps humain. Le dioxygène y est notamment absorbé par l'organisme tandis que le dioxyde de carbone est rejeté dans l'air.

Les poumons rassemblent l'ensemble des alvéoles pulmonaires. Afin de pouvoir assurer leur fonction, la surface d'échange avec le milieu intérieur est particulièrement grande dans ces organes. Cette spécificité fait des poumons une zone privilégiée pour l'absorption de molécules thérapeutiques.

Le nez est tapissé de muqueuses riches en vaisseaux sanguins. La muqueuse est notamment recouverte de mucus nasal, qui piège les molécules entrantes et permet leur présentation aux terminaisons nerveuses à des fins d'olfaction. Le nez est aussi pourvu d'un système ciliaire dont le rôle est de filtrer l'air inspiré pour éviter de l'inspiration d'organismes ou de molécules étrangères.

Il convient donc de comprendre que le nez constitue un obstacle à la propagation de molécules thérapeutiques vers les poumons, et de ce fait, l'inhalation d'un médicament doit se faire préférentiellement par respiration buccale. Cependant, l'inhalation nasale est un réflexe, et il est donc difficile de contraindre le patient à inspirer par la bouche, *a fortiori* lorsque le patient est un enfant ou un bébé.

A titre d'exemple, un masque facial pour inhalation classique tel que celui décrit dans la demande de brevet EP 1 389 482 est un masque respiratoire oro-nasal, c'est-à-dire qui entoure la bouche et le nez lorsqu'il est appliqué sur le visage du patient. Il présente une unique cavité et une ouverture permettant l'abouchement d'un élément sur le masque tel qu'une source de diffusion d'O₂. Ce masque permet donc de favoriser la respiration du patient, mais se révèle peu adapté à l'inhalation de molécules thérapeutiques du fait que celles-ci ne peuvent pas être dirigées préférentiellement vers l'une ou l'autre des entrées du système respiratoire. La demande de brevet WO 2012/173 992 divulgue un masque facial pour inhalation présentant une partie logeant le nez et une partie logeant la bouche du patient ainsi qu'une ouverture alignée avec le nez permettant l'inhalation de molécules thérapeutiques par le patient. Cette solution favorise l'inhalation des molécules thérapeutiques par le nez, ce qui est un obstacle à la déposition optimale de molécules thérapeutiques dans les poumons du patient.

La demande de brevet US 2005/217 678 décrit un masque facial pour inhalation présentant une pièce intra-buccale. De ce fait, la contrainte d'inspiration par la bouche est assurée, mais la pièce intra-buccale génère un réel inconfort pour le patient, et se révèle particulièrement inadaptée lorsque ce patient est un nourrisson.

La demande de certificat d'utilité CN 203090169 U présente un masque respiratoire permettant de rassurer l'enfant et de lui administrer des médicaments. Le masque comprend un tuyau pénétrant dans la cavité buccale du patient, ce qui n'est cependant pas une solution pleinement confortable notamment chez l'enfant et le nourrisson.

Le brevet américain No. US 5,243,972 divulgue un masque respiratoire antifumée, mais ce masque n'est pas constitué d'une seule pièce moulée et une lèvre retournée vers l'intérieur du corps telle que dans la présente invention.

Le brevet américain No. US 1,310,825 divulgue un appareil respiratoire constitué d'une seule pièce moulée, mais l'appareil englobe la totalité du visage de l'utilisateur.

La demande de brevet Internationale WO 2010/076715 divulgue un appareil respiratoire constitué d'une seule pièce moulée ou une lèvre retournée vers l'intérieur du corps, mais ledit appareil ne comprend pas deux chambres.

La demande de brevet Américaine No. US 2013/0255678 divulgue un nébuliseur pour enfant ou pour patients ayant des difficultés respiratoires qui est formé d'une seule pièce moulée, mais qui ne comprend pas pas deux chambres telle que dans la présente invention.

La demande de brevet Américaine No. US 2002/0170557 divulgue un masque avec une valve d'inhalation constitué d'une seule pièce moulée et comportant une lèvre retournée vers l'intérieur du corps, mais le masque ne comprend pas deux chambres.

La demande de brevet No. Internationale WO 99/21602 divulgue un masque avec divulgue un assemblage de masque facial constitué d'une seule pièce moulée, mais il ne divulgue pas un masque comprenant deux chambres telle que dans la présente invention.

Le brevet américain No. US 5,265,595 divulgue un masque pour l'analyse de la respiration constitué d'une seule pièce moulée ou une lèvre retournée vers l'intérieur du corps, mais ce document n'enseigne pas une première lèvre recourbée vers l'intérieur du corps bordant le pourtour de la seconde ouverture.

Actuellement, il existe donc des masques d'inhalation contraignant le patient à respirer par la bouche par l'intermédiaire de pièces buccales. Ces dispositifs sont néanmoins inconfortables pour le patient et particulièrement inadaptés pour les jeunes patients, ce qui gêne l'inhalation du médicament et incommode le patient. En outre, de tels dispositifs sont complexes à fabriquer et/ou à utiliser.

Le but de l'invention est de pallier les inconvénients précités. En particulier, un des buts de l'invention est de proposer un masque facial pour inhalation conçu pour permettre une inhalation et une déposition pulmonaire des molécules thérapeutiques optimales sans générer de contraintes fortes ou de gêne lors de l'utilisation du masque par le patient.

En particulier, un but de l'invention est de proposer un tel masque permettant une inhalation du gaz ou de la molécule thérapeutique uniquement par la bouche.

Un autre but de l'invention est de proposer un tel masque afin de réduire le risque de refus du patient d'utiliser le masque.

Un autre but de l'invention est de proposer un tel masque qui soit simple de conception et d'utilisation.

### RÉSUMÉ

A cet effet, la présente invention concerne un masque comprenant un corps dont au moins une paroi délimite un volume intérieur destiné à engager une portion du visage d'un utilisateur incluant son nez et sa bouche, le corps présentant une première ouverture en communication avec un moyen de raccordement à une source de gaz ou d'une molécule thérapeutique, et une seconde ouverture bordée par une lèvre destinée à être appliquée contre ladite portion du visage de l'utilisateur, caractérisé en ce qu'il comporte une cloison étanche délimitant, dans le volume intérieur, une chambre supérieure destinée à loger le nez de l'utilisateur et une chambre inférieure contenant ladite première ouverture et destinée à loger la bouche de l'utilisateur lorsque ledit masque est porté.

Le masque englobe à la fois le nez et la bouche du patient. La cloison permet de séparer de manière étanche la chambre recevant le nez, de la chambre recevant la bouche de l'utilisateur. Seule la chambre recevant la bouche met en communication les voies respiratoires de l'utilisateur avec la source générant le gaz respiratoire ou la molécule thérapeutique par l'intermédiaire du moyen de raccordement R1. L'inhalation de l'individu est donc contrainte de se faire uniquement par la bouche. Selon un mode de réalisation de l'invention, la cloison étanche est bordée par une seconde lèvre destinée à être appliquée au-dessus de la lèvre supérieure de l'utilisateur. Avantageusement, la seconde lèvre se confond au niveau de ses extrémités avec la première lèvre.

Les lèvres du masque ainsi jointes permettent d'assurer l'étanchéité du pourtour labial et du nez. Cette caractéristique renforce l'étanchéité entre les deux chambres et participe à contraindre l'inhalation du gaz ou de la molécule thérapeutique par la bouche. Le masque est constitué d'une seule pièce moulée ergonomiquement par exemple selon un procédé de moulage par injection. La chambre supérieure comprend une valve unidirectionnelle expiratoire.

La valve unidirectionnelle expiratoire est destinée à permettre la sortie de l'air expiré par le patient, à interdire l'entrée d'air dans cette partie du masque et a pour but d'améliorer le confort du patient. La valve permet de favoriser l'effort inspiratoire du patient par la bouche.

Selon un mode de réalisation de l'invention, la lèvre présente une forme globale trilobique.

Le masque de section trilobique est spécifiquement adapté pour les très jeunes enfants, puisque chez cette population, la distance nez-bouche est très réduite. Cette adaptation du modèle du masque permet notamment de limiter les échanges gazeux directement entre la chambre nasale et l'air ambiant.

Selon un mode de réalisation de l'invention, la lèvre présente une forme globale piriforme.

Le masque à la lèvre piriforme convient particulièrement aux adultes et aux enfants. En effet, ces individus présentent des particularités physionomiques au niveau de la sphère oro-nasale, et plus particulièrement en ce qui concerne la distance nezbouche, mais la distance est plus grande que celle observée pour les nourrissons et très jeunes enfants. Le masque de ce type permet donc de prendre en compte ces impératifs du visage de l'utilisateur, en assurant notamment l'étanchéité du contact entre les lèvres du masque et le visage.

Selon un mode de réalisation de l'invention, le masque est réalisé dans un matériau plastique ou dans un matériau élastomère.

Lorsque le matériau plastique est un élastomère, l'utilisation d'un matériau à base de silicone est privilégiée. La silicone a pour intérêt d'être aussi neutre que possible d'un point de vue biologique, ce qui en fait un matériau idéal pour la fabrication d'un élément destiné à être en contact avec le corps humain. Il existe néanmoins sur le marché des élastomères de synthèse qui possèdent aussi toutes les caractéristiques nécessaires à l'élaboration d'un tel masque. De tels élastomères sont bien entendu envisagés dans le cadre de la présente invention.

L'invention concerne encore un dispositif d'inhalation tel que décrit précédemment comprenant un masque d'inhalation tel que décrit précédemment et une chambre d'inhalation ou un nébuliseur, la chambre d'inhalation et le nébuliseur étant raccordés au moyen de raccordement du masque.

La présente invention a pour objet de présenter un masque, pour l'administration d'un gaz ou d'une molécule thérapeutique, constitué d'une seule pièce moulée destinée à engager la portion du visage d'un utilisateur incluant son nez et sa bouche, ladite pièce comprenant un corps dont au moins une paroi délimite un volume intérieur, le corps comportant une cloison étanche délimitant, dans le volume intérieur, une chambre supérieure destinée à loger le nez de l'utilisateur, la chambre supérieure comprenant une valve unidirectionnelle expiratoire, et une chambre inférieure destinée à loger la bouche de l'utilisateur lorsque ledit masque est porté, le corps présentant une première ouverture en communication avec un moyen de raccordement à une source de gaz ou d'une molécule thérapeutique, le moyen de raccordement débouchant dans la chambre inférieure sans pénétrer dans la bouche, et une seconde ouverture bordée par une première lèvre recourbée vers l'intérieur du corps et destinée à être appliquée contre ladite portion du visage de l'utilisateur.

Le masque d'inhalation selon la présente invention peut être caractérisé en ce que la cloison étanche est bordée par une seconde lèvre destinée à être appliquée au-dessus de la lèvre supérieure de l'utilisateur.

Le masque d'inhalation selon la présente invention peut être caractérisé en ce que la seconde lèvre se confond au niveau de ses extrémités avec la première lèvre.

Le masque d'inhalation selon la présente invention peut être caractérisé en ce que la première lèvre, bordant ladite seconde ouverture destinée à être appliquée contre ladite portion du visage de l'utilisateur, présente une forme globale trilobique.

Le masque d'inhalation selon la présente invention peut être caractérisé en ce que la première lèvre, bordant ladite seconde ouverture destinée à être appliquée contre ladite portion du visage de l'utilisateur, présente une forme globale piriforme.

Le masque selon la présente invention peut être caractérisé en ce qu'il est réalisé dans un matériau plastique ou dans un matériau élastomère.

La présente invention a pour objet de présenter, selon un autre aspect de l'invention un dispositif d'inhalation, caractérisé en ce qu'il comprend un masque d'inhalation selon la présente invention et une chambre d'inhalation ou un nébuliseur, la chambre d'inhalation étant branchée au moyen de raccordement.

Les caractéristiques de l'invention mentionnées ci-dessus, ainsi que d'autres, apparaîtront plus clairement à la lecture de la description suivante d'un exemple de réalisation.

### BRÈVE DESCRIPTION DES DESSINS

L'invention sera mieux comprise en se référant aux dessins annexés, lesquels sont cependant donnés à simple but illustratif et sans l'intention de limiter la portée de l'invention. Dans les dessins :
La Fig. 1 présente une vue en perspective avant d'un masque facial pour inhalation selon un mode de réalisation ne faisant pas partie de l'invention,
La Fig. 2 présente une vue en perspective arrière d'un masque facial pour inhalation selon un mode de réalisation ne faisant pas partie de l'invention,
La Fig. 3 présente une vue en coupe médiane d'un masque facial pour inhalation selon un mode de réalisation ne faisant pas partie de l'invention,
La Fig. 4 présente une vue arrière d'un masque facial pour inhalation selon un mode de réalisation ne faisant pas partie de l'invention,
La Fig. 5 présente une vue arrière d'une première variante de réalisation d'un masque facial pour inhalation adapté à la physionomie faciale d'un nourrisson selon un mode de réalisation ne faisant pas partie de l'invention,
Les Figs. 6 et 7 représentent deux vues arrière de deux masques d'inhalation selon les Figs. 4 et 5, et pourvus d'une valve d'expiration, selon des modes de réalisation de l'invention.
La Fig. 8 représente une vue d'un masque facial pour inhalation selon un mode de réalisation ne faisant pas partie de l'invention, couplé à une chambre d'inhalation.

On notera que dans les dessins annexés, les éléments similaires sont identifiés par des numéros de référence similaires.

### DESCRIPTION DÉTAILÉE

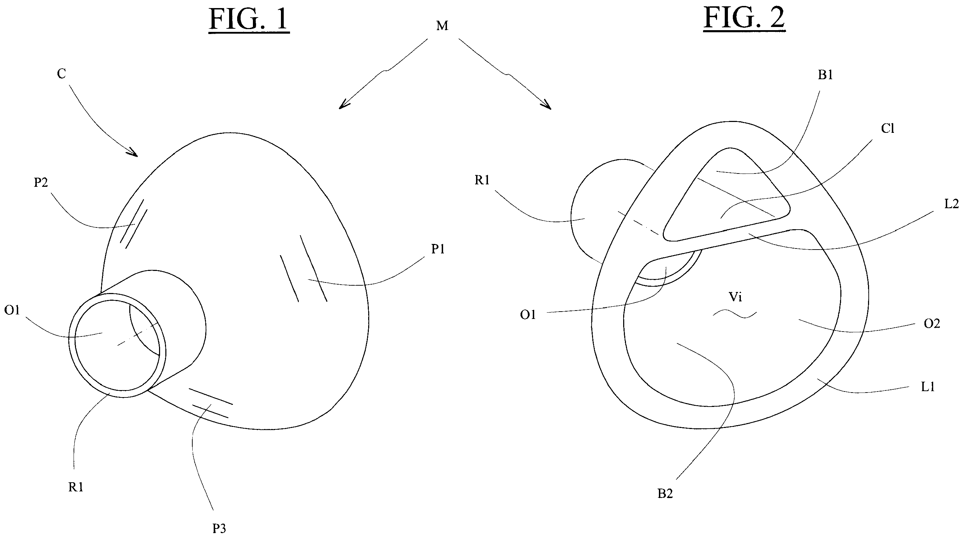

Le dispositif présenté en lien avec les Figs. 1 à 7 se rapporte à un masque (M) comprenant un corps (C) dont au moins une paroi (P1, P2, P3) délimite un volume intérieur (Vi) destiné à engager une portion du visage d'un utilisateur incluant son nez et sa bouche, le corps (C) présentant une première ouverture (O1) en communication avec un moyen de raccordement (R1) à une source de gaz ou d'une molécule thérapeutique, et une seconde ouverture (O2) bordée par une lèvre (L1) destinée à être appliquée contre ladite portion du visage de l'utilisateur, caractérisé en ce qu'il comporte une cloison étanche (C1) délimitant, dans le volume intérieur (Vi), une chambre supérieure (B1) destinée à loger le nez de l'utilisateur et une chambre inférieure (B2) contenant ladite première ouverture (O1) et destinée à loger la bouche de l'utilisateur lorsque ledit masque est porté.

Il s'agit d'un masque facial pour inhalation, adapté à l'administration d'un gaz ou d'une molécule thérapeutique dans le cadre d'un traitement par inhalation. Le masque facial pour inhalation selon l'invention est, en particulier mais de manière non limitative, conçu pour être utilisé par un enfant, un nourrisson et/ou un prématuré.

Sur les Figs. 1 à 7, le masque facial pour inhalation M comprend un corps C présentant un volume Vi débouchant par une ouverture O1 dans un moyen de raccordement R1 vers un dispositif, tel qu'un dispositif d'inhalation, un nébuliseur ou toute autre source générant un gaz ou une molécule thérapeutique sous forme volatile.

Dans le mode de réalisation représenté en lien avec les Figs. 1, 2, 3 et 4, le corps C a la forme globale d'une section de pyramide à trois faces et aux arêtes courbées. Il est ainsi délimité globalement par trois parois P1, P2 et P3. Ce mode de réalisation est particulièrement pertinent pour la réalisation d'un masque selon l'invention qui réponde aux impératifs morphologiques d'un adulte ou d'un enfant.

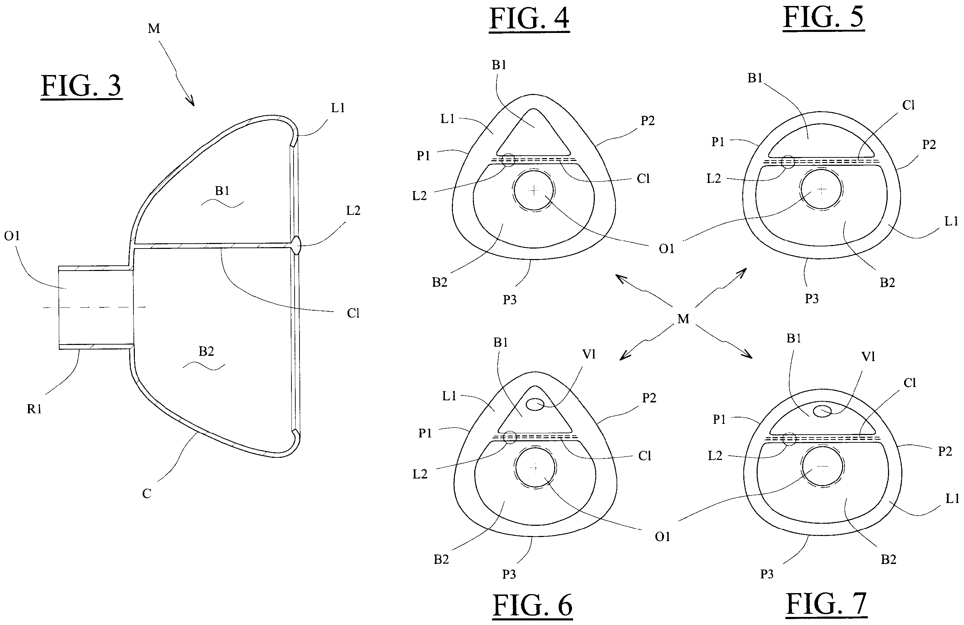

Dans les modes de réalisation représentés en lien avec les Figs. 5 et 7, le corps C a la forme globale d'une section de sphère. Elle est ainsi délimitée globalement par trois parois identiques P1, P2 et P3. Ce mode de réalisation est particulièrement pertinent pour la réalisation d'un masque qui réponde aux impératifs morphologiques d'un nourrisson ou d'un prématuré.

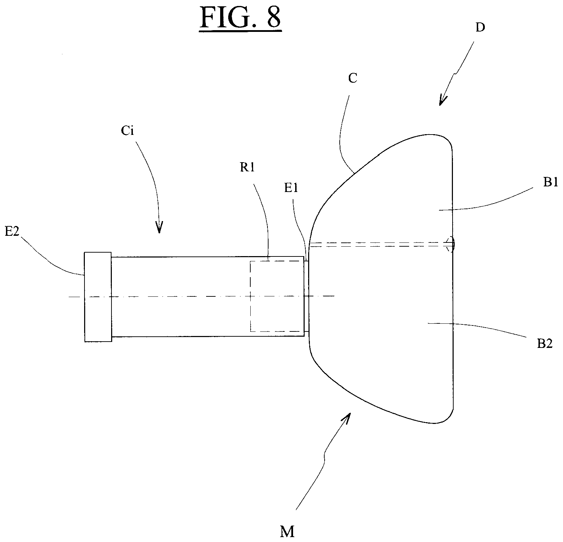

Le moyen de raccordement R1 consiste dans ces modes de réalisation, en une portion de cylindre creux permettant l'abouchement du masque avec un dispositif complémentaire, tel qu'une chambre d'inhalation comme représenté sur la Fig. 8 ou encore un nébuliseur.

Comme cela est visible à la Fig. 2, le corps C est délimité par une ouverture 02 bordée d'une lèvre L1 destinée à être appliquée autour de la sphère oro-nasale de l'utilisateur. La lèvre L1 est recourbée vers l'intérieur du corps C du masque M, de telle sorte que le bord libre de la lèvre L1 n'est pas en contact avec l'utilisateur du masque.

La lèvre L1 du masque facial pour inhalation M représenté sur la Fig. 4 présente sur sa vue arrière une géométrie piriforme. Elle est particulièrement adaptée à des enfants dont la distance nez-bouche commence à augmenter significativement.

De manière différente, la Fig. 5 présente une version du masque de l'invention, adaptée à des utilisateurs dont la distance nez-bouche est plus réduite, c'est-à-dire adaptée aux nourrissons et aux prématurés. La lèvre L1 de ce masque présente sur sa vue arrière de la Fig. 5, une géométrie trilobique.

On observe sur les Figs. 2 à 7, la présence d'une cloison C1, délimitant à l'intérieur du corps C du masque M, deux chambres, l'une supérieure B1 et l'autre inférieure B2. La chambre supérieure B1 est conçue pour recevoir le nez de l'utilisateur alors que la chambre inférieure B2 est prévue pour recevoir la bouche de l'utilisateur lorsque le masque M est porté. La cloison C1 s'étend, comme cela est visible sur les Figs. 2 et 4 à 7, sur toute la largeur du masque, de la paroi P1 à la paroi P2.

On peut observer sur la Fig. 3 que la cloison C1 s'étend également sur toute la profondeur du masque. Elle se raccorde, dans la position de pose du masque par une personne en station debout, au-dessus du moyen de raccordement R1.

Le bord libre de la cloison C1 est délimité par une seconde lèvre L2 (Fig. 3). La lèvre L2 est destinée à être mise en contact avec le visage de l'utilisateur et précisément au dessus de sa lèvre supérieure, et en déport de ses narines.

La Fig. 3 permet également d'observer que la seconde lèvre L2 se raccorde par ses deux extrémités avec la lèvre L1. En conséquence, les lèvres L1 et L2 sont en continuité. Ceci procure une étanchéité sur le pourtour labial et autour du nez. La chambre supérieure B1 ne comprend que le nez, alors que la chambre inférieure B2 ne comprend que la bouche de l'utilisateur du masque. Les chambres B1 et B2 sont séparées de manière étanche l'une de l'autre.

Le moyen de raccordement R1 communique avec la chambre buccale B2 du masque facial pour inhalation M. L'inhalation est ainsi contrainte de se faire exclusivement par la bouche de l'utilisateur. Elle est réalisée sans tubage susceptible de pénétrer dans la bouche. L'utilisation du masque M est particulièrement facile, surtout pour soigner des enfants en bas âge et les nourrissons.

Selon une autre variante présentée sur les Figs. 6 et 7, une valve expiratoire V1 est disposée au niveau d'une ouverture pratiquée dans la chambre supérieure ou chambre nasale B1. Il s'agit d'une valve unidirectionnelle qui est positionnée au niveau d'une paroi arrondie de jonction entre la paroi P1 et la paroi P2. Elle permet à l'utilisateur du masque d'expulser hors du masque et sans gêne l'air contenu dans ses poumons.

Le masque de l'invention n'entraîne aucune gêne du fait de son utilisation, notamment au niveau de la cavité buccale.

Il permet de réduire le risque de refus du patient d'utiliser le masque.

Il facilite son utilisation par le personnel soignant, qu'ils soient professionnels ou non professionnels.

Sur la Fig. 8 est représenté un dispositif d'inhalation D. Un masque facial pour inhalation M, tel que l'un de ceux décrits dans les Figs. 1 à 7, est connecté par l'intermédiaire de son moyen de raccordement R1 à une chambre d'inhalation Ci. Une source générant un gaz ou une molécule thérapeutique, telle qu'un flacon diffuseur d'aérosol, peut être connectée à la seconde extrémité E2 de cette chambre d'inhalation pour permettre la diffusion de la molécule ou du gaz à l'intérieur de la chambre d'inhalation Ci lors du traitement et après actionnement de l'aérosol. Lors de la phase d'inspiration/inhalation provoquée par le patient, la molécule thérapeutique va diffuser de la chambre d'inhalation Ci à la chambre inférieure B2 du masque M par l'intermédiaire du moyen de raccordement R1. La cloison C1 va empêcher la molécule thérapeutique ou le gaz d'atteindre la chambre supérieure B1, n'autorisant ainsi l'inhalation de la molécule thérapeutique ou du gaz que par la bouche de l'utilisateur.

| **Références** | **Désignation** | **Fig.** |
|---|---|---|
| M | Masque facial pour inhalation | 1 2 3 4 5 6 7 8 |
| C | Corps | 1 3 8 |
| R1 | Moyen de raccordement (tube) | 1 2 3 8 |
| P1 P2 P3 | Parois | 1 4 5 6 7 |
| L1 | Lèvre | 2 3 4 5 6 7 |
| L2 | Seconde lèvre | 2 3 4 5 6 7 |
| C1 | Cloison | 2 3 4 5 6 7 |
| B1 B2 | Chambres | 2 3 4 5 6 7 8 |
| V1 | Valve expiratoire | 6 7 |
| Ci | Chambre d'inhalation | 8 |
| Vi | Volume intérieur | 2 |
| O1 | Première ouverture | 1 2 3 4 5 6 7 |
| O2 | Seconde ouverture | 2 |
| D | Dispositif d'inhalation | 8 |

## Revendications

1. Masque (M), pour l'administration d'un gaz ou d'une molécule thérapeutique, constitué d'une seule pièce moulée destinée à engager la portion du visage d'un utilisateur incluant son nez et sa bouche, ladite pièce comprenant un corps (C) dont au moins une paroi (P1, P2, P3) délimite un volume intérieur (Vi), le corps (C) comportant une cloison étanche (C1) délimitant, dans le volume intérieur (Vi), une chambre supérieure (B1) destinée à loger le nez de l'utilisateur, la chambre supérieure (B1) comprenant une valve unidirectionnelle expiratoire (V1), et une chambre inférieure (B2) destinée à loger la bouche de l'utilisateur lorsque ledit masque est porté, le corps (C) présentant une première ouverture (O1) en communication avec un moyen de raccordement (R1) à une source de gaz ou d'une molécule thérapeutique, le moyen de raccordement (R1) débouchant dans la chambre inférieure (B2) sans pénétrer dans la bouche, et une seconde ouverture (O2) bordée par une première lèvre (L1) recourbée vers l'intérieur du corps (C) et destinée à être appliquée contre ladite portion du visage de l'utilisateur.

2. Masque (M) d'inhalation selon la revendication 1, **caractérisé en ce que** la cloison étanche (C1) est bordée par une seconde lèvre (L2) destinée à être appliquée au-dessus de la lèvre supérieure de l'utilisateur.

3. Masque (M) d'inhalation selon la revendication 2, **caractérisé en ce que** la seconde lèvre (L2) se confond au niveau de ses extrémités avec la première lèvre (L1).

4. Masque (M) d'inhalation selon l'une quelconque des revendications 1 à 3, **caractérisé en ce que** la première lèvre (L1), bordant ladite seconde ouverture (O2) destinée à être appliquée contre ladite portion du visage de l'utilisateur, présente une forme globale trilobique.

5. Masque (M) d'inhalation selon l'une quelconque des revendications 1 à 3, **caractérisé en ce que** la première lèvre (L1), bordant ladite seconde ouverture (O2) destinée à être appliquée contre ladite portion du visage de l'utilisateur, présente une forme globale piriforme.

6. Masque selon l'une quelconque des revendications 1 à 5, **caractérisé en ce qu'**il est réalisé dans un matériau plastique ou dans un matériau élastomère.

7. Dispositif d'inhalation, **caractérisé en ce qu'**il comprend un masque (M) d'inhalation tel que décrit dans l'une quelconque des revendications 1 à 6 et soit une chambre d'inhalation (Ci) étant branchée au moyen de raccordement (R1), soit un nébuliseur.

## Patentansprüche

1. Maske (M) zur Abgabe eines therapeutischen Gases oder Moleküls, bestehend aus einem einzigen Formteil, das bestimmt ist, den Abschnitt des Gesichts eines Benutzers inkl. seiner Nase und seines Mundes zu umschließen, wobei das Teil einen Körper (C) umfasst, von dem mindestens eine Wand (P1, P2, P3) ein Innenvolumen (Vi) begrenzt, wobei der Körper (C) eine dichte Trennwand (C1) umfasst, die in dem Innenvolumen (Vi) eine obere Kammer (B1) begrenzt, die zur Aufnahme der Nase des Benutzers bestimmt ist, wobei die obere Kammer (B1) ein eindirektionales Atemventil (V1) umfasst, und eine untere Kammer (B2), die zur Aufnahme des Mundes des Benutzers bestimmt ist, wenn die Maske getragen wird, wobei der Körper (C) eine erste Öffnung (O1) in Kommunikation mit einem Anschlussmittel (R1) an eine Quelle therapeutischen Gases oder Moleküls aufweist, wobei das Anschlussmittel (R1) in die untere Kammer (B2) ausmündet, ohne in den Mund einzudringen, und eine zweite Öffnung (O2), die von einer in das Innere des Körpers (C) gekrümmten ersten Lippe (L1) gesäumt ist und bestimmt, am Abschnitt des Gesichts des Benutzers anzuliegen.

2. Inhalationsmaske (M) nach Anspruch 1, **dadurch gekennzeichnet, dass** die dichte Trennwand (C1) von einer zweiten Lippe (L2) gesäumt ist, die bestimmt ist, oberhalb der Oberlippe des Benutzers anzuliegen.

3. Inhalationsmaske (M) nach Anspruch 2, **dadurch gekennzeichnet, dass** die zweite Lippe (L2) im Bereich ihrer Enden in die erste Lippe (L1) übergeht.

4. Inhalationsmaske (M) nach einem der Ansprüche 1 bis 4, **dadurch gekennzeichnet, dass** die erste Lippe (L1), die die zweite Öffnung (O2) säumt, die bestimmt ist, am Abschnitt des Gesichts des Benutzers anzuliegen, eine allgemeine dreilappige Form aufweist.

5. Inhalationsmaske (M) nach einem der Ansprüche 1 bis 4, **dadurch gekennzeichnet, dass** die erste Lippe (L1), die die zweite Öffnung (O2) säumt, die bestimmt ist, am Abschnitt des Gesichts des Benutzers anzuliegen, eine allgemeine Birnenform aufweist.

6. Maske nach einem der Ansprüche 1 bis 5, **dadurch gekennzeichnet dass** sie aus einem Kunststoffmaterial oder aus einem Elastomermaterial hergestellt ist.

7. Inhalationsvorrichtung, **dadurch gekennzeichnet, dass** sie eine Inhalationsmaske (M) nach einem der Ansprüche 1 bis 6 und entweder eine Inhalationskammer (Ci), die an das Anschlussmittel (R1) angeschlossen ist, oder einen Vernebeler umfasst.

## Claims

1. A mask (M), for administering a gas or a therapeutic molecule, made as a single moulded piece for engaging the portion of a user's face including his/her nose and mouth, said piece comprising a body (C) at least one wall (P1, P2, P3) of which delimits an inner volume (Vi), the body (C) including a sealed partition (C1) delimiting, in the inner volume (Vi), an upper chamber (B1) for accommodating the user's nose, the upper chamber (B1) comprising a unidirectional expiratory valve (VI), and a lower chamber (B2) for accommodating the user's mouth when said mask is worn, the body (C) having a first opening (O1) in communication with a connecting means (R1) for connecting to a gas or a therapeutic molecule source, the connecting means (R1) opening into the lower chamber (B2) without penetrating the mouth, and a second opening (O2) bordered by a first lip (L1) bent inwardly of the body (C) and for being applied against said portion of the user's face.

2. The inhalation mask (M) according to claim 1, **characterised in that** the sealed partition (C1) is bordered by a second lip (L2) for being applied above the user's upper lip.

3. The inhalation mask (M) according to claim 2, **characterised in that** the second lip (L2) is merged with the first lip (L1) at its ends.

4. The inhalation mask (M) according to any of claims 1 to 3, **characterised in that** the first lip (L1), bordering said second opening (O2) for being applied against said portion of the user's face, has overall a trilobal shape.

5. The inhalation mask (M) according to any of claims 1 to 3, **characterised in that** the first lip (L1), bordering said second opening (O2) for being applied against said portion of the user's face, has overall a piriform shape.

6. The mask according to any of claims 1 to 5, **characterised in that** it is made of a plastic material or elastomeric material.

7. An inhalation device, **characterised in that** it comprises an inhalation mask (M) as described in any of claims 1 to 6 and either an inhaling chamber (Ci) being connected to the connecting means (R1), or a nebuliser.
